Europäisches Patentamt

European Patent Office

Office européen des brevets

(19)

(11) Veröffentlichungsnummer: **0 068 194**
**B1**

(12) # EUROPÄISCHE PATENTSCHRIFT

(45) Veröffentlichungstag der Patentschrift:
16.01.85

(51) Int. Cl.⁴: **C 07 C 11/02,** C 07 C 7/148,
C 07 C 7/12 // C07C41/06

(21) Anmeldenummer: 82105007.7

(22) Anmeldetag: 08.06.82

(54) Verfahren zur Isolierung von reinen tert.-Olefinen aus Kohlenwasserstoffgemischen.

(30) Priorität: 19.06.81 DE 3124294

(43) Veröffentlichungstag der Anmeldung:
05.01.83 Patentblatt 83/1

(45) Bekanntmachung des Hinweises auf die Patenterteilung:
16.01.85 Patentblatt 85/3

(84) Benannte Vertragsstaaten:
DE FR GB IT NL

(56) Entgegenhaltungen:
EP - A - 0 003 305
DE - A - 2 938 222

(73) Patentinhaber: BAYER AG, Konzernverwaltung RP
Patentabteilung, D-5090 Leverkusen 1 Bayerwerk (DE)
Patentinhaber: EC ERDÖLCHEMIE GMBH,
Postfach 75 20 02, D-5000 Köln 71 (DE)

(72) Erfinder: Herwig, Jens, Dr., Auf dem Hügel 23,
D-5000 Köln 41 (DE)
Erfinder: Schulwitz, Bruno, Dr.,
Luetzlongericherstrasse 64, D-5000 Köln 60 (DE)
Erfinder: Schleppinghoff, Bernhard, Dr., Adolf Kolping
Strasse 5, D-4047 Dormagen 1 (DE)
Erfinder: Scheef, Hans-Volker, Goethestrasse 69,
D-4047 Dormagen 1 (DE)
Erfinder: Lange, Peter Michael, Dr.,
Walter-Flex-Strasse 9, D-5090 Leverkusen (DE)

**Beschreibung**

Die vorliegende Erfindung betrifft ein Verfahren zur Isolierung von reinen $C_4$ bis $C_{10}$-tert.-Olefinen aus mindestens ein $C_4$ bis $C_{10}$-tert.-Olefin enthaltenden Kohlenwasserstoffgemischen durch Veretherung mit einem $C_1$ bis $C_4$-Alkanol und nachfolgende Etherspaltung unter Freisetzung der reinen $C_4$ bis $C_{10}$-tert.-Olefine, wobei die Hauptmenge des bei der Etherspaltung freiwerdenden $C_1$ bis $C_4$-Alkanols destillativ von den $C_4$ bis $C_{10}$-tert.-Olefinen abgetrennt wird und die in die Olefinfraktion gelangte Restalkanolmenge durch Absorption an einem Absorberharz entfernt wird und wobei das in die Veretherung einzusetzende $C_1$ bis $C_4$-Alkanol teilweise dem Sumpf der Alkanolabtrennung und teilweise durch Desorption dem mit dem $C_1$ bis $C_4$-Alkanol beladenen Absorberharz entnommen wird.

Tertiäre Olefine sind wichtige Vorprodukte zur Herstellung von Polymeren und höherwertigen Chemikalien. So erlaubt die interessante Funktion der Doppelbindung am tertiären Kohlenstoffatom die Erzeugung zahlreicher organischer Zwischenprodukte, wie beispielsweise Pinakolin und Neocarbonsäuren oder durch Dehydrierung die Erzeugung konjugierter Diolefine, wie beispielsweise Isopren, die im Kunststoff-, Pharma-, Pflanzenschutz- und Schmierstoffsektor weiterverarbeitet werden können. Die Voraussetzung für derartige Umsetzungen liegt in der Verfügbarkeit solcher tertiärer Olefine in möglichst hoher Reinheit.

Da diese tertiären Olefine hauptsächlich im Gemisch mit anderen Kohlenwasserstoffen im Produktspektrum von thermischen und katalytischen Crackern anfallen, muss zu ihrer Reindarstellung ein Isolierungsverfahren angewendet werden. Zur Isolierung der tertiären Olefine aus den genannten Kohlenwasserstoffströmen diente bisher in erster Linie das Schwefelsäureverfahren, bei dem zunächst die Schwefelsäureester der den tertiären Olefinen entsprechenden tertiären Alkohole gebildet wurden, die anschliessend durch Abspaltung der Schwefelsäure wieder zu den tertiären Olefinen zurückgebildet werden. Dieses Verfahren ist durch die Korrosivität der Schwefelsäure und durch die notwendige Aufarbeitung der Abfallschwefelsäure stark belastet. In neuerer Zeit sind Verfahren entwickelt worden, die — ausgehend von einer selektiven Veretherung der tertiären Olefine — mit einem Alkanol, die Reingewinnung der tertiären Olefine über die Zersetzung dieser Ether erreichen (EP-Anmeldung Nr. 0003305; US Nr. 3170000; DE-OS Nr. 2924869). Die Trennung der aus der Etherspaltung kommenden Produkte in tert.-Olefin und Alkanol erfolgt im allgemeinen durch Destillation, bei der die tert.-Olefine als Kopfprodukt anfallen und das Alkanol in den Sumpf geht. Aufgrund azeotroper Effekte enthält das Kopfprodukt jedoch häufig noch Alkohol, das durch eine Wasserwäsche vom tertiären Olefin getrennt werden kann, wenn ein reines tertiäres Olefin erhalten werden soll. Hierbei erhält man eine wässerige Lösung des Alkanols, die durch Destillation wieder aufgearbeitet werden kann. Für eine derartige Abtrennung ist jedoch ein zusätzlicher Energieaufwand für die Destillationsstufe erforderlich. Ausserdem wird je nach Einsatzgebiet des Olefins gegebenenfalls eine Trocknung an einem Molekularsieb zur $H_2O$-Entfernung erforderlich.

Die Herstellung von Alkyl-tert.-alkylethern, die in das reine tert.-Olefin und das zugehörige Alkanol gespalten werden sollen, ist ebenfalls bekannt. Sie kann durch Umsetzung von Gemischen, die mindestens ein tert.-Olefin neben anderen Olefinen und Paraffinkohlenwasserstoffen enthalten, mit einem Alkanol in Gegenwart von sauren Katalysatoren durchgeführt werden. Als saure Katalysatoren werden beispielsweise anorganische Säuren, wie Schwefelsäure, oder organische Säuren, wie p-Toluolsulfonsäure oder saure, sulfonsäuregruppenhaltige Kationenaustauscher auf der Basis vernetzter vinylaromatischer Polymere verwendet (DE-AS Nr. 1224294). Zur Vervollständigung der Etherbildung und zur Abfuhr der Reaktionswärme kann ein Teil des Reaktionsgemisches aus dem Olefin und einem Alkanol direkt in das Einsatzprodukt für den Herstellungsreaktor zurückgeführt werden (DE-OS Nr. 2911077). Ein höherer Olefinumsatz kann auch durch den Einsatz mehrerer nachgeschalteter Reaktoren erreicht werden.

Es wurde nun ein Verfahren zur Isolierung von reinen $C_4$ bis $C_{10}$-tert.-Olefinen aus mindestens ein $C_4$ bis $C_{10}$-tert.-Olefin enthaltenden Kohlenwasserstoffgemischen durch Umsetzung mit einem $C_1$ bis $C_4$-Alkanol zu den entsprechenden Alkyl-tert.-alkylethern, Abtrennung der restlichen, im wesentlichen von den $C_4$ bis $C_{10}$-tert.-Olefinen freien Kohlenwasserstoffe von den Alkyl-tert.-alkylethern, Rückspaltung der Alkyl-tert.-alkylether in die $C_4$ bis $C_{10}$-tert.-Olefine und das $C_1$ bis $C_4$-Alkanol und Abtrennung der Hauptmenge des bei der Etherspaltung freiwerdenden $C_1$ bis $C_4$-Alkanols von den $C_4$ bis $C_{10}$-tert.-Olefinen durch Destillation gefunden, das dadurch gekennzeichnet ist, dass die restliche, in die Fraktion der $C_4$ bis $C_{10}$-tert.-Olefine gelangte Menge an $C_1$ bis $C_4$-Alkanol aus dieser Fraktion durch Absorption an einem synthetischen Ionenaustauscher mit Kationen oder Anionen austauschenden Gruppen oder einem Gemisch hieraus entfernt wird und das zur Umsetzung zu den Alkyl-tert.-alkylethern erforderliche $C_1$ bis $C_4$-Alkanol teilweise durch Desorption des Alkanols vom Absorberharz mit Hilfe der mindestens ein tert.-Olefin enthaltenden Kohlenwasserstoffgemische bereitgestellt wird.

Als Alkanol für das erfindungsgemässe Verfahren sei ein primärer oder sekundärer Alkohol mit 1 bis 4 C-Atomen genannt, wie Methanol, Ethanol, Propanol, i-Propanol, Butanol, i-Butanol, bevorzugt Methanol oder Ethanol, besonders bevorzugt Methanol.

Als tert.-Olefine, die erfindungsgemäss in reiner Form hergestellt werden können, seien Olefine mit einer Verzweigung der Kohlenstoffkette an der olefinischen Doppelbindung mit 4 bis 10, bevorzugt 4 bis 8, besonders bevorzugt 4 bis 6 C-Atomen genannt, wie i-Buten, tert.-Penten, tert.-Hexen, tert.-Octen oder tert.-Decen. In ganz besonders bevorzugter Weise seien das i-Buten und die Isoamylene genannt.

Das in reiner Form herzustellende tert.-Olefin wird in das erfindungsgemässe Verfahren in Form eines Kohlenwasserstoffgemisches eingeführt, das mindestens ein solches tert.-Olefin enthält. Solche Stoffströme enthalten neben dem mindestens einen tert.-Olefin weitere Olefine oder Paraffinkohlenwasserstoffe, gegebenenfalls zusätzlich Wasserstoff, Stickstoff, CO oder $CO_2$. Beispiele für solche Stoffströme sind: ein Roh-$C_4$-Schnitt oder ein $C_4$-Raffinat I (nach der Butadienextraktion) aus einem thermischen Cracker oder ein FCC-Rohproduktstrom aus einem *fluid-catalytic-cracker* (FCC) oder ein $C_4/C_5$-LCC-Strom eines katalytischen Crackers (LCC = *light catalytic cracker*). Selbstverständlich kann der FCC-Rohproduktstrom vorher in einen $C_4$-FCC-Strom und einen $C_5$-FCC-Strom aufgeteilt worden sein. Ebenso geeignet ist ein $C_5$-Strom eines thermischen Crackers, beispielsweise ein Destillationsvorlauf bei der Aromatengewinnung. Weitere geeignete Produktströme sind solche einer n-Butenskelettisomerisierung oder einer i-Butandehydrierung, in denen sich i-Buten vorfindet. Als Einsatzströme zur Umsetzung höherer tert.-Olefine eignen sich beispielsweise Destillatschnitte der jeweiligen Kohlenstoffzahlfraktionen oder in analoger Weise, wie für das i-Buten skizziert, Dehydrierungs- oder Skelettisomerisierungsströme von Kohlenwasserstoffen mit den entsprechenden C-Zahlen. Für den Fall, dass Kohlenwasserstoffgemische, die mehr als 1 tert.-Olefin enthalten, eingesetzt werden, können die Gemische mehrerer Alkyl-tert.-alkylether entstehen. Diese können in der nachfolgenden Etherspaltung dann die Gemische mehrerer tert.-Olefine liefern, die dann in reiner Form vorliegen und dadurch leichter in die Einzelkomponenten aufgetrennt werden können. Selbstverständlich können auch für den Fall, dass Gemische mehrerer Alkyl-tert.-alkylether entstehen, diese Ether vor der Spaltung in die reinen Produkte getrennt werden, so dass nach der Spaltung der reinen Ether auch die reinen Einzelkomponenten der tert.-Olefine gewonnen werden.

Als Verhältnis des Alkanols zu dem oder den tert.-Olefinen sei beispielsweise 0,8 bis 5 mol Alkanol pro Mol tert.-Olefin oder pro 1 mol der Molsumme verschiedener tert.-Olefine genannt. Für den Fall der Veretherung des i-Butens, insbesondere mit Methanol, sei bevorzugt der Überschussbereich des Alkanols, beispielsweise 1 bis 4, bevorzugt 1,1 bis 3 mol Alkanol pro Mol i-Buten genannt. Für den Fall höherer tert.-Olefine, deren Gleichgewichtspunkt zur Umwandlung in den zugehörigen Ether vom 100% igen Umsatz deutlich abweicht, kann auch der Unterschussbereich des Alkanols bevorzugt sein. Hier sei beispielsweise ein Verhältnis von 0,8 bis 2, besonders bevorzugt 0,9 bis 1,5 mol Alkanol pro Mol höheres tert.-Olefin, genannt.

Die Veretherung im Rahmen des erfindungsgemässen Verfahrens findet in an sich bekannter Weise an einem sauren Veretherungskontakt statt, beispielsweise in Gegenwart einer starken Mineralsäure oder deren Anhydrid, wie Schwefelsäure, Phosphorsäure, Schwefeltrioxid, Oleum oder Phosphorpentoxid, oder an einem stark sauren Ionenaustauscher, wie einem sulfonsäurehaltigen Styrol/Divinylbenzol-Polymerisat oder einem sulfonsäurehaltigen Formaldehyd/Phenol-Harz.

Als Temperatur sei beispielsweise der Bereich von 25 bis 90, bevorzugt von 45 bis 75°C, genannt. Die volumetrische Stundenraumgeschwindigkeit in Liter Kohlenwasserstoff-Einsatzstrom pro Liter sauren Kontaktes pro Stunde sei beispielsweise ein Wert von 0,05 bis 50, bevorzugt von 0,2 bis 15, genannt. Der Druck für die Veretherung wird so gewählt, dass die am Verfahren beteiligten Stoffe in flüssiger Form vorliegen. Als Druckbereich sei allgemein der Bereich von 0,2 bis 30, bevorzugt von 2 bis 20 bar, genannt. Der Druck im unteren Bereich ab 0,2 bar kommt hierbei nur für tert.-Olefine und die Kohlenwasserstoffgemische, in denen diese tert.-Olefine enthalten sind, sowie für die zur Veretherung gelangenden Alkanole in Betracht, die unter diesen Druckbedingungen noch flüssig sind.

Der Veretherungsstufe schliesst sich sodann eine Abtrennung des gebildeten Alkyl-tert.-alkylethers vom nichtveretherten Restkohlenwasserstoffgemisch mit den nichtveretherbaren Olefinen und den Paraffinkohlenwasserstoffen sowie gegebenenfalls restlichem tert.-Olefin, das wegen der Lage des Veretherungsgleichgewichts nicht umgesetzt wurde, an.

Gegebenenfalls kann der Ether in einer anschliessenden Destillation von höheren Kohlenwasserstoffen und höheren Ethern feindestilliert werden.

Die nachfolgende Rückspaltung des Alkyl-tert.-alkylethers oder, für den Fall, dass mehrere tert.-Olefine simultan umgesetzt werden sollen, des Gemisches solcher Alkyl-tert.-alkylether, kann beispielsweise an kristalliner Kieselsäure im Bereich von etwa 190 bis 350°C durchgeführt werden (DE-OS Nr. 2924869). Die Etherspaltung kann jedoch auch nach dem Verfahren der EP-Anmeldung Nr. 0054805 an sauren Molekularsieben, wie Zeolithen, sauren Aluminiumoxiden oder H-Mordeniten, durchgeführt werden, die gegebenenfalls durch eine Behandlung mit Wasserstoff aktiviert worden sind.

Solche sauren Molekularsiebe besitzen beispielsweise ein effektives Porenvolumen von beispielsweise 0,05 bis 0,6 ml/g und einen effektiven Porendurchmesser von beispielsweise 3 bis 15 Å. Ihre spezifische Oberfläche beträgt etwa 100 bis 700 m²/g. Dieses letztere Verfahren kann bei einer Temperatur von etwa 100 bis 300°C in der Gasphase oder in der Flüssigphase bei einem Druck von etwa 0,1 bis 50 bar durchgeführt werden. Die Kontaktbelastung wird im Bereich von WHSV = 0,5 bis 5 g Substrat pro Gramm Katalysator pro Stunde gewählt (WHSV = *weight, hourly space velocity*).

Eine Aktivierung mit Wasserstoff kann beispielsweise mit 50 bis 500 I Wasserstoff pro Liter Katalysator bei etwa 100 bis 450°C und 1 bis 50 bar für etwa 1 bis 60 h durchgeführt werden.

Bei dieser Etherspaltung erhält man sodann ein Gemisch des zugrundeliegenden Alkanols und des tert.-Olefins.

Das Gesamtverfahren der Herstellung von reinen tert.-Olefinen in der beschriebenen Art kann durch die folgende Gleichung wiedergegeben werden

$$\begin{matrix} \text{Einsatzstrom} & \text{Alkanol} \\ \text{mit} & + \text{(ggf. Über-} \rightarrow \\ \text{tert.-Olefin} & \text{schuss)} \end{matrix}$$

$$\begin{matrix} & \text{Alkyl-} & \text{Reststrom,} \\ \rightarrow & \text{tert.-} & + \text{weitgehend frei} & \text{(I)} \\ & \text{alkylether} & \text{von tert.-Olefin} \end{matrix}$$

$$\begin{matrix} \text{Alkyl-tert.-} & \rightarrow & \text{tert.-Olefin (rein) +} \\ \text{alkylether} & & \text{Alkanol} \end{matrix} \quad \text{(II)}$$

Erfindungsgemäss werden die auftretenden restlichen Mengen an Alkanol, die nach der Rückspaltung des Alkyl-tert.-alkylethers und der in an sich bekannter Weise durchgeführten destillativen Abtrennung der Hauptmenge des Alkanols in die dabei entstandene Olefinfraktion gelangt sind, durch Absorption an einem Absorberharz zurückgewonnen. In bevorzugter Weise beträgt die Hauptmenge des Alkanols 60 bis 99 Gew.-% der nach der Etherspaltung vorhandenen Gesamtmenge, so dass der verbleibende Teil von 1 bis 40 Gew.-% in erfindungsgemässer Weise durch Absorption an einem Absorberharz zurückgewonnen werden. Weiterhin wird erfindungsgemäss dieses auf dem Absorberharz absorbierte Alkanol zur Bildung des Alkyl-tert.-alkylethers derart bereitgestellt, dass der Einsatz-Kohlenwasserstoffstrom mit mindestens einem tert.-Olefin zur Desorption dieses Alkanols durch das mit dem Alkanol beladene Absorberharz geleitet wird. Ferner wird diesem Strom das destillativ abgetrennte Alkanol zugegeben und, falls erforderlich, durch Frischalkanol ergänzt und in den Veretherungsreaktor geleitet.

Die oben dargestellten Formulierungen des Verfahrens lassen sich somit für die erfindungsgemässe Arbeitsweise wie folgt darstellen

$$\begin{matrix} \text{Einsatzstrom} \\ \text{mit} & + \begin{matrix}\text{Absorberharz,} \\ \text{alkanolbeladen}\end{matrix} & + \begin{matrix}\text{abdestilliertes} \\ \text{Alkanol} \rightarrow\end{matrix} \\ \text{tert.-Olefin} \end{matrix}$$

$$\rightarrow \begin{matrix}\text{Alkyl-tert.-} \\ \text{alkylether}\end{matrix} + \begin{matrix}\text{Reststrom,} \\ \text{weitgehend frei} \\ \text{von tert.-Olefin}\end{matrix} + \begin{matrix}\text{Absor-} \\ \text{berharz,} \\ \text{unbeladen}\end{matrix} \quad \text{(III)}$$

$$\begin{matrix}\text{Alkyl-} \\ \text{tert.-} \\ \text{alkylether}\end{matrix} \rightarrow \begin{matrix}\text{tert.-Olefin} \\ \text{(alkanolhaltig)}\end{matrix} + \begin{matrix}\text{abdestilliertes} \\ \text{Alkanol}\end{matrix}$$

$$\begin{matrix}\text{tert.-} \\ \text{Olefin} \\ \text{(alkanol-} \\ \text{haltig)}\end{matrix} + \begin{matrix}\text{Absor-} \\ \text{berharz,} \\ \text{unbeladen}\end{matrix} \rightarrow \begin{matrix}\text{tert.-} \\ \text{Olefin} \\ \text{(rein)}\end{matrix} + \begin{matrix}\text{Absor-} \\ \text{berharz,} \\ \text{alkanol-} \\ \text{beladen}\end{matrix} \quad \text{(IV)}$$

Aus der formelähnlichen Darstellung wird ersichtlich, dass das gesamte zur Veretherung eingesetzte Alkanol in Form von wiedergewonnenem destilliertem Alkanol und in Form von durch die Etherspaltung zurückgewonnenem Alkanol vollständig zurückerhalten wird. Abgesehen von in einem technischen Verfahren unvermeidlichen Leckagen und von kleinen Restmengen Alkanol, die entweder im reinen tert.-Olefin oder im weitgehend vom tert.-Olefin befreiten Reststrom vorhanden sind, ist somit keine Zuführung von Alkanol zum erfindungsgemässen Verfahren erforderlich. Vielmehr wird die im Rahmen der beschriebenen Verfahrensgegebenheiten einmal eingesetzte Alkanolmenge, ergänzt um die erwähnten geringen Leckage- und sonstigen Verluste, ständig im Kreis gefahren. Als im Kreislauf gefahrene Alkanolmenge seien 0,8 bis 5 mol pro Mol tert.-Olefin oder pro Mol der Molsumme verschiedener tert.-Olefine genannt. Geht man weiterhin aus von der Tatsache, dass die Gesamtmenge an gebildetem Alkyl-tert.-alkylether in der Etherrückspaltung wieder verbraucht wird, kann das gesamte erfindungsgemässe Verfahren zur Herstellung von reinen tert.-Olefinen zusammengefasst durch die folgende Formulierung dargestellt werden

$$\begin{matrix} \text{Einsatzstrom} & \text{(Benutzung von} & \text{tert.-Olefin} \\ \text{mit} & \text{Absorberharz)} & \text{(rein)} \\ \text{tert.-Olefin} & \xrightarrow{\hspace{2cm}} \end{matrix}$$

$$+ \begin{matrix}\text{Reststrom,} \\ \text{weitgehend frei} \\ \text{von tert.-Olefin}\end{matrix} \quad \text{(V)}$$

Das zur Absorption und Desorption eingesetzte Absorberharz wird zur Durchführung des erfindungsgemässen Verfahrens in mindestens zwei Schichten aufgeteilt, von denen mindestens eine in weitgehend alkanolfreiem Zustand zur Absorption des bei der Etherrückspaltung auftretenden Alkanols eingesetzt wird, während eine andere bereits mit dem Alkanol beladene Absorberschicht in den Strom der Einsatzstoffe für das erfindungsgemässe Verfahren zur Desorption des Alkanols geschaltet wird. Gegebenenfalls ist es erforderlich, eine weitere Absorberharzschicht nach der Bildung des Ethers zur Absorption des überschüssigen Alkanols aus dem vom tert.-Olefin befreiten Reststrom zu schalten, die dann ebenfalls zur Rückführung dieses dabei aufgefangenen Alkanols wieder in den Einsatzstrom zur Desorption geschaltet wird. Die zur Absorption und zur Desorption vorgesehenen Absorberharzschichten werden durch einfache und dem Fachmann geläufige Verschaltung so kombiniert, dass rechtzeitig vor dem Durchschlagen des Alkanols durch die Absorberschicht diese Absorberschicht zur Desorption umgeschaltet wird und eine durch Desorption inzwischen weitgehend alkanolfrei gewordene Absorberharzschicht nunmehr neu Alkanolabsorption zur Verfügung steht. Dieses Umschalten der verschiedenen Absorberharzschichten von Absorption auf Desorption kann durch simultane Betätigung aller dazu erforderlichen Absperrorgane so schnell ausgeführt werden, dass das erfindungsgemässe Verfahren vollkontinuierlich durchgeführt werden kann. Selbstverständlich kann man zur Alkanolabsorption auch mehr als eine Absorberschicht einsetzen, wobei hinter die erste Absorberschicht eine zweite als Sicherheitsschicht geschaltet wird. Hierbei kann die Kapazität für das Alkanol in der ersten Absorberschicht voll ausgenutzt werden, da ein teilweises Durchschlagen des Alkanols durch die erste Schicht in der zweiten Schicht aufgefangen wird. Das Durchschlagen kann in einfacher Weise, beispielsweise durch gaschromatographische Messung oder durch Infrarotdetektoren im austretenden Kohlenwasserstoffstrom, erkannt werden.

Die Absorption des überschüssigen Alkanols kann beispielsweise im Bereich von 0 bis 60, bevorzugt 15 bis 35°C, durchgeführt werden. Als Druck kann beispielsweise 0,2 bis 20 bar gewählt werden. Zur Absorption befinden sich die vom Alkanol zu befreienden Stoffströme im flüssigen Zustand. Der untere Teil des angegebenen Druckbereiches kommt daher nur in Frage, wenn höhere tert.-Olefine und/oder höhere Alkanole umgesetzt werden, die sich ebenso wie die daraus gebildeten Alkyl-tert.-alkylether auch unter vermindertem Druck noch in flüssigem Zustand befinden.

Die Desorption des Alkanols mit Hilfe des Einsatzstromes kann grundsätzlich unter den gleichen Bedingungen ablaufen wie die Absorption. Im allgemeinen ist es wünschenswert, die Desorptionszeit etwa gleich gross wie die Absorptionszeit zu halten, um einerseits bei Sättigung des zur Absorption eingesetzten Harzes eine regenerierte Schicht zur Verfügung zu haben, andererseits das Alkanol möglichst gleichmässig in die Veretherungsreaktion abzugeben. Hierzu kann die Desorption auch in einem etwas erhöhten Temperaturbereich, beispielsweise 20 bis 100, bevorzugt 30 bis 60°C, und einem Druck von beispielsweise 3 bis 30, bevorzugt 5 bis 15 bar, durchgeführt werden. Der Gesamtbereich für die Desorption beträgt demnach 0,2 bis 30 bar bei 0 bis 100°C. Überraschenderweise wurde beobachtet, dass mit Hilfe des Einsatzstromes eine um so raschere Desorption erreicht werden kann, je höher der Gehalt an tert.-Olefinen darin ist. Dies erlaubt eine Fahrweise, bei der die Desorptionstemperatur niedrig, beispielsweise in der Nähe der Absorptionstemperatur, gehalten werden kann. Dies ergibt eine äusserst energiesparende Fahrweise, die in energetischer Hinsicht den bisher bekannten Verfahren zur Abtrennung von Methanol, nämlich einer Wasserwäsche mit nachfolgender destillativer Aufarbeitung des Alkanol/Wasser-Gemisches, überlegen ist.

Als Absorberharze für das erfindungsgemässe Verfahren seien beispielsweise synthetische Ionenaustauscher mit Kationen oder Anionen austauschenden Gruppen genannt. Solche Ionenaustauscher haben beispielsweise eine Matrix auf der Basis von vernetzten Styrolpolymeren. Als Vernetzer seien beispielsweise Divinylbenzol, Trivinylbenzol oder Trivinylcyclohexan in einer Menge von etwa 0,3 bis 80, bevorzugt 1 bis 65, besonders bevorzugt 2 bis 50 Gew.-%, bezogen auf die Gesamtmenge der Comonomeren, genannt. Die Matrix kann jedoch auch ein Phenol/Formaldehyd-Kondensat, ein (Meth)Acrylharz oder ein Epichlorhydrin/Polyamin-Kondensat, jeweils in vernetzter Form, sein. Solche vernetzten Matrizen können in der gelförmigen oder der makroporösen Form eingesetzt werden.

Als austauschende Gruppen auf diesen Matrizen seien beispielsweise genannt: Sulfonsäuregruppen, Phosphorsäuregruppen und Carboxylgruppen, jeweils in der $H^+$-Form oder beispielsweise in der $Na^+$-, $K^+$-, $NH_4^+$- oder $Ca^{++}$-Form. Weitere austauschende Gruppen können beispielsweise sein: $-NR_3^+$, wie $-N(CH_3)_3^+$ oder $-N(CH_3)_2CH_2CH_2OH^+$, oder $NR_2$, wie $N(CH_3)_2$, sowie N-Oxidgruppen. Solche N-haltigen Gruppen können als austauschbares Gegenion beispielsweise das $OH^-$, $Cl^-$ oder $SO_4$-Ion enthalten. Solche Anionaustauscher mit der Gruppe $-N(CH_3)_3^+$ sind dem Fachmann als solche des Typs I und mit der Gruppe $-N(CH_3)_2CH_2CH_2OH^+$ als solche des Typs II bekannt.

Ionenaustauscher der beschriebenen Arten haben beispielsweise Totalkapazitäten für den Ionenaustausch von etwa 0,5 bis 6 Val/l Harz. Solche Harze und ihre Herstellungsverfahren sind seit langem bekannt (Hefferich, „Ion Exchange", McGraw-Hill, New York [1962]).

Beispiele für erfindungsgemäss einsetzbare Harze sind: Sulfonsäuregruppen enthaltende gelförmige Styrol/Divinylbenzol-Harze, Sulfonsäuregruppen enthaltende makroporöse Styrol/Divinylbenzol-Harze, Carboxylgruppen enthaltende gelförmige oder makroporöse (Meth)Acrylsäure/Divinylbenzol-Harze, gelförmige oder makroporöse Styrol/Divinylbenzol-Anionenaustauscher von einem der Typen I oder II, makroporöse oder gelförmige Styrol/Divinylbenzol-Harze mit $-N(CH_3)_2$-Gruppen, schwach basische, makroporöse Harze des Acrylamid/Divinylbenzol-Typs, stark basische, makroporöse Harze des Acrylamid/Divinylbenzol-Typs oder Sulfonsäuregruppen enthaltende vernetzte Phenol/Formaldehyd-Harze. Die Aufzählung ist nicht vollständig und stellt keine Einschränkung auf die genannten Harze dar. Viele dieser Harze sind Handelsprodukte verschiedener Hersteller.

Die Belastung der Absorberschicht zur Absorption und zur Desorption wird innerhalb des Bereiches einer stündlichen Raumgeschwindigkeit WHSV von 0,1 bis 100, bevorzugt 0,5 bis 20, besonders bevorzugt von 1 bis 15 kg Einsatzstrom pro Kilo Absorber pro Stunde gewählt.

Eine Abtrennung von nicht umgesetztem Alkanol der Veretherungsstufe ist nicht unbedingt erforderlich, da bezogen auf den Alkyl-tert.-alkylether das gleiche Alkanol bei seiner Spaltung entsteht. Alkanolmengen, die in den weitgehend vom tert.-Olefin befreiten Reststrom gelangt sind, können gegebenenfalls auch absorptiv entfernt und zurückgeführt werden.

Es ist selbstverständlich, dass durch unvollständige Umsetzungen übriggebliebene Wertstoffe, gegebenenfalls nach Isolierung, in die einzelnen Stufen des erfindungsgemässen Verfahrens zurückgeführt werden können. So kann beispielsweise nicht vollständig umgesetztes tert.-Olefin im Reststrom, insbesondere bei den tert.-Olefinen mit höherer C-Zahl, beispielsweise durch Destillation abgetrennt werden und erneut mit dem Einsatzstrom zusammen in die Veretherungsstufe eingeführt werden. Weiterhin kann gegebenenfalls nicht vollständig rückgespaltener Ether bei der Isolierung des reinen tert.-Olefins und der Abtrennung des Rückspaltalkanols aufgefangen werden und die Etherspaltung erneut eingesetzt werden. Ebenso selbstverständlich ist es, unvermeidliche Verunreinigungen, wie unerwünschte Zersetzungsprodukte oder Oligomerisierungsprodukte, durch Ausschleusung kleiner Teilströme aus den im Verfahren auftretenden Teilströmen zu eliminieren und deren Konzentration in allen Teilströmen möglichst niedrig zu halten. Hierbei mit ausgeschleuste kleine Alkanolmengen, deren Wiedergewinnung wirtschaftlich nicht sinnvoll ist, werden, wie bereits erwähnt, durch Zusatz von Frisch-Alkanol ersetzt.

*Beispiel*

Zur Veretherung eines teilhydrierten $C_5$-Kohlenwasserstoffstromes eines thermischen Crackers (Aromatenvorlauf) mit Methanol wird ein temperierbarer Durchlaufreaktor mit einer lichten Weite von 20 mm eingesetzt. Die Temperaturkontrolle erfolgt über Temperaturmessstellen, welche im Abstand von 100 mm angeordnet sind. In diesem Reaktor werden 100 g/h des genannten Kohlenwasserstoffstromes mit 20 Gew.-% i-Amylen und 8,6 g/h Methanol an 110 g eines mit 0,75 g/l elementarem Palladium dotierten stark sauren makroporösen Kationenaustauschers nach DE-OS Nr. 3036481 bei 70°C und 10 bar verethert. Die gaschromatographische Untersuchung des Produktstromes weist folgende Zusammensetzung auf

|                                    | Gew.-% |
|------------------------------------|--------|
| i-Amylene                          | 4,1    |
| TAME (tert.-Amylmethylether)       | 20,9   |
| Methanol                           | 1,2    |
| Rest $C_5$-Kohlenwasserstoffe      | 72,1   |
| Höhere Ether und Kohlenwasser-stoffe | 1,7  |

Umsatz an i-Amylen: 78 Gew.-% des eingesetzten i-Amylens.

Dieser Produktstrom wird in einem Zwischenbehälter aufgefangen und von dort in eine Destillationseinrichtung gegeben. Als Kopfprodukt dieser Destillation wird der $C_5$-Kohlenwasserstoffstrom mit den Restmengen an i-Amylen und Methanol abgezogen, als Sumpfprodukt wird TAME im Gemisch mit höheren Ethern entnommen. Dieses Sumpfprodukt wird in einer weiteren Destillationskolonne auf reines TAME aufgearbeitet (als Kopfprodukt mit 99,9 Gew.-% TAME). Als Sumpfprodukt dieser Reindestillation werden höhere Ether als unerwünschte Produkte ausgeschleust.

Das gereinigte TAME wird in einen Spaltreaktor gefahren, in welchem das TAME in das i-Amylen und Methanol rückgespalten wird.

Der Spaltreaktor ist ein temperierbarer Durchlaufreaktor mit einem lichten Durchmesser von 25 mm, ausgerüstet mit Temperaturmessstellen (Abstand 100 mm) und einer regelbaren Druckhaltung. Die Zudosierung des TAME erfolgt über eine Membrankolbenpumpe.

Die Reaktionsbedingungen
Katalysator: 100 g $H^+$-Mordenit
Einsatz: 100 g TAME/h (99,9 Gew.-% Reinheit)
WHSV: 1 g Substrat pro Gramm Kontakt pro Stunde
Druck: 10 bar
Temperatur: 140°C
Umsatz TAME: 96%

Die gaschromatographische Analyse des Spaltproduktes ergibt folgende Zusammensetzung

|                  | Gew. -% |
|------------------|---------|
| TAME             | 3,8     |
| 2-Methylbuten-2  | 49,2    |
| 2-Methylbuten-1  | 16,8    |
| 3-Methylbuten-1  | –       |
| Methanol         | 28,0    |
| Dimethylether    | 1,6     |
| $H_2O$           | 0,6     |

Dieses Spaltprodukt wird in einem auf etwa 0°C gekühlten Zwischenbehälter gelagert und in einer nachgeschalteten Destillationsstufe in die gewünschten Methylbutene und restliches TAME und Methanol aufgetrennt. In dieser Destillation erhält man als Kopfprodukt

|                  | Gew.-% |
|------------------|--------|
| 3-Methylbuten-1  | <0,1   |
| 2-Methylbuten-2  | 71,1   |
| 2-Methylbuten-1  | 24,3   |
| Methanol         | 4,6    |
| TAME             | <0,1   |

Die Zusammensetzung dieses Kopfproduktes entspricht dem Azeotrop i-Amylenmethanol. Der Methanolgehalt im Sumpf dieser Destillation liegt bei mehr als 82 Gew.-%.

Zur Methanolentfernung und Reindarstellung der i-Amylene wird das Kopfprodukt über einen Absorber gefahren. Dieser Absorber besteht aus zwei im Wechseltakt betriebenen Stahlreaktoren mit einem lichten Durchmesser von 25 mm und einem Gesamtvolumen von 250 ml. Die Reaktoren sind mantelbeheizt und mit Temperaturmessstellen versehen. Der Reaktordruck wird über eine Druckhaltung geregelt. Die Zudosierung des Einsatzstromes erfolgt von unten nach oben über eine Membrankolbenpumpe. Am Ausgang des Reaktors werden Moment- und Durchschnittsproben gezogen und gaschromatographisch untersucht. Die im Wechseltakt betriebenen Reaktoren sind mit je 120 ml eines makroporösen Styrol/Divinylbenzol-Anionenaustauschers mit Dimethylbenzylamingruppen (Handelsprodukt Lewatit MP 62 der Bayer AG) gefüllt. Zur Methanolabsorption werden 400 g/h des Kopfproduktes der beschriebenen Destillation von unten nach oben über einen der beiden Absorber geleitet. Der Druck wird bei 1 bar und die Temperatur bei 20°C gehalten. Die Absorptionseffekte und ihre zeitliche Entwicklung sind in der folgenden Tabelle wiedergegeben

| Produktdurchsatz | CH$_3$OH-Konzentration am Absorberausgang (Gew.-%) |
|---|---|
| 400 g | 0,1 |
| 800 g | 0,1 |
| 1200 g | 0,1 |
| 1600 g | 0,2 |

Anschliessend wird der Produktstrom auf den zweiten Absorptionsreaktor umgeschaltet, der in gleicher Weise betrieben wird. Der beladene Absorber wird zur Desorption des Methanols mit dem Einsatzstrom, welcher verethert werden soll, gespült. Die Reaktionsbedingungen hierfür sind p = 5 bar; T = 35°C; Durchsatz = 440 g/h.

Die Methanolkonzentration im Eluat und ihre Entwicklung über 3 h sind in der nachfolgenden Tabelle aufgelistet

| Produktdurchsatz | CH$_3$OH-Konzentration Gew.-% |
|---|---|
| 440 g | 8,6 |
| 880 g | 2,3 |
| 1332 g | 0,1 |

Der Absorptionsreaktor wird auf 20°C abgekühlt und der Reaktordruck auf 1 bar abgesenkt. In dieser Form ist der Absorber für die nächste Taktumschaltung zur Methanolabsorption betriebsbereit.


**Patentansprüche**

1. Verfahren zur Isolierung von reinen C$_4$ bis C$_{10}$-tert.-Olefinen aus mindestens ein C$_4$ bis C$_{10}$-tert.-Olefin enthaltenden Kohlenwasserstoffgemischen durch Umsetzung mit einem C$_1$ bis C$_4$-Alkanol zu den entsprechenden Alkyltert.-alkylethern, Abtrennung der restlichen, im wesentlichen von den C$_4$ bis C$_{10}$-tert.-Olefinen freien Kohlenwasserstoffe von den Alkyl-tert.-alkylethern, Rückspaltung der Alkyl-tert.-alkylether in die C$_4$ bis C$_{10}$-tert.-Olefine und das C$_1$ bis C$_4$-Alkanol und Abtrennung der Hauptmenge des bei der Etherspaltung freiwerdenden C$_1$ bis C$_4$-Alkanols von den C$_4$ bis C$_{10}$-tert.-Olefinen durch Destillation, dadurch gekennzeichnet, dass die restliche, in die Fraktion der C$_4$ bis C$_{10}$-tert.-Olefine gelangte Menge an C$_1$ bis C$_4$-Alkanol aus dieser Fraktion durch Absorption an einem synthetischen Ionenaustauscher mit Kationen oder Anionen austauschenden Gruppen oder einem Gemisch hieraus entfernt wird und das zur Umsetzung zu den Alkyl-tert.-alkylethern erforderliche C$_1$ bis C$_4$-Alkanol teilweise durch Desorption des Alkanols vom Absorberharz mit Hilfe der mindestens ein tert.-Olefin enthaltenden Kohlenwasserstoffgemische bereitgestellt wird.

2. Verfahren nach Anspruch 1, dadurch gekennzeichnet, dass als Absorberharz ein Ionenaustauscher mit einer Matrix aus Styrol/Divinylbenzol, Phenol/Formaldehyd, (Meth)Acrylsäure (derivat)/Divinylbenzol oder Epichlorhydrin/Polyamin eingesetzt wird.

3. Verfahren nach den Ansprüchen 1 und 2, dadurch gekennzeichnet, dass das C$_1$ bis C$_4$- Alkanol nach der Rückspaltung des Alkyl-tert.-alkylethers zu 60 bis 99 Gew.-% in bekannter Weise vom C$_4$ bis C$_{10}$-tert.-Olefin destillativ getrennt wird.

4. Verfahren nach den Ansprüchen 1 bis 3, dadurch gekennzeichnet, dass pro Mol C$_4$ bis C$_{10}$-tert.-Olefin oder pro Mol der Molsumme verschiedener C$_4$ bis C$_{10}$-tert.-Olefine 0,8 bis 5 mol C$_1$ bis C$_4$-Alkanol teilweise als durch Destillation rückgewonnenes C$_1$ bis C$_4$-Alkanol und teilweise als vom Absorberharz durch Desorption gewonnenes C$_1$ bis C$_4$-Alkanol in das mindestens ein C$_4$ bis C$_{10}$-tert.-Olefin enthaltende Kohlenwasserstoffgemisch eingespeist werden.

5. Verfahren nach den Ansprüchen 1 bis 4, dadurch gekennzeichnet, dass die Alkanolabsorption bei 0 bis 60°C und 0,2 bis 20 bar durchgeführt wird.

6. Verfahren nach den Ansprüchen 1 bis 4, dadurch gekennzeichnet, dass die Alkanoldesorption bei 0 bis 100°C und 0,2 bis 30 bar durchgeführt wird.

7. Verfahren nach den Ansprüchen 1 bis 6, dadurch gekennzeichnet, dass die Belastung der Absorberschicht auf 0,1 bis 100 kg Einsatzstrom pro Kilo Absorber pro Stunde eingestellt wird.


**Claims**

1. Process for the isolation of pure C$_4$ to C$_{10}$-tert.-olefines from hydrocarbon mixtures containing at least one C$_4$ to C$_{10}$-tert.-olefine by reaction with a C$_1$ to C$_4$-alkanol to give the corresponding alkyl tert.-alkyl ethers, separating off the residual hydrocarbons which are largely free of the C$_4$ to C$_{10}$-tert.-olefines, from the alkyl tert.-alkyl ethers, cleavage of the alkyl tert.-alkyl ethers back to the C$_4$ to C$_{10}$-tert.-olefines and the C$_1$ to C$_4$-alkanol and separation of the main quantity of the C$_1$ to C$_4$-alkanol liberated during the ether cleavage from the C$_4$ to C$_{10}$-tert.-olefines by distillation, characterised in that the residual quantity of C$_1$ to C$_4$-alkanol which has entered the fraction of the C$_4$ to C$_{10}$-tert.-olefines is removed from this fraction by absorption on to a synthetic ion exchanger with groups which exchange cations or anions or a mixture of these, and the C$_1$ to C$_4$-alkanol necessary for the reaction to give the alkyltert.-alkyl ethers is at least partly made available by desorption of the alkanol from the absorber resin with the aid of the hydrocarbon mixtures containing at least one tert.-olefine.

2. Process according to Claim 1, characterised in that the absorber resin used is an ion exchanger with a matrix composed of styrene/divinylbenzene, phenyl/formaldehyde, (meth)acrylic acid (derivative)/divinylbenzene or epichlorohydrin/polyamine.

3. Process according to Claims 1 and 2, characterised in that the C$_1$ to C$_4$-alkanol, after the back-cleavage of the alkyl tert.-alkyl ether, is separated by distillation from the C$_4$ to C$_{10}$-tert.-olefine to an extent of 60 to 99% by weight, in a known manner.

4. Process according to Claims 1 to 3, characterised in that per mol of $C_4$ to $C_{10}$-tert.-olefine or per mol of the molar sum of several different $C_4$ to $C_{10}$-tert.-olefines 0.8 to 5 mol of $C_1$ to $C_4$-alkanol are fed, partly in the form of $C_1$ to $C_4$-alkanol recovered by distillation and partly in the form of $C_1$ to $C_4$-alkanol recovered from the absorber resin by desorption, into the hydrocarbon mixture containing at least one $C_4$ to $C_{10}$-tert.-olefine.

5. Process according to Claims 1 to 4, characterised in that the alkanol absorption is carried out at 0 to 60°C and under 0.2 to 20 bar.

6. Process according to Claims 1 to 4, characterised in that the alkanol desorption is carried out at 0 to 100°C and under 0.2 to 30 bar.

7. Process according to Claims 1 to 6, characterised in that the loading of the absorber layer is set at 0.1 to 100 kg of inlet stream per kilo of absorber per hour.

## Revendications

1. Procédé pour isoler à l'état pur des oléfines tertiaires en $C_4$ à $C_{10}$ de mélanges d'hydrocarbures contenant au moins une oléfine tertiaire en $C_4$ à $C_{10}$, par réaction avec un alcanol en $C_1$ à $C_4$ pour former les éthers d'alkyle et d'alkyle tertiaire correspondants par séparation des hydrocarbures résiduels, principalement dépourvus des oléfines tertiaires en $C_4$ à $C_{10}$, des éthers d'alkyle et d'alkyle tertiaire, par rétroscission des éthers d'alkyle et tertioalkyle en les oléfines tertiaires en $C_4$ à $C_{10}$ et en l'alcanol en $C_1$ à $C_4$ et par séparation de la majeure partie de l'alcanol en $C_1$ à $C_4$ libéré de la coupure de l'éther des oléfines tertiaires en $C_4$ à $C_{10}$ par distillation, caractérisé en ce que la quantité résiduelle d'alcanol en $C_1$ à $C_4$ parvenue dans la fraction des oléfines tertiaires en $C_4$ à $C_{10}$ est éliminée de cette fraction par absorption sur un échangeur ionique synthétique portant des groupes d'échange avec des cations ou des anions ou leur mélange, et en ce que l'alcanol en $C_1$ à $C_4$ nécessaire pour la réaction avec les éthers d'alkyle et de tertioalkyle est tenu prêt en partie par désorption de l'alcanol de la résine absorbante à l'aide des mélanges d'hydrocarbures contenant au moins une oléfine tertiaire.

2. Procédé suivant la revendication 1, caractérisé en ce qu'on utilise comme résine absorbante un échangeur d'ions à matrice de styrène/divinylbenzène, phénol/formaldéhyde, (dérivé d')acide acrylique (méthacrylique)/divinylbenzène ou épichlorhydrine/polyamine.

3. Procédé suivant les revendications 1 et 2, caractérisé en ce que l'alcanol en $C_1$ à $C_4$ est séparé par distillation, d'une manière connue, de l'oléfine tertiaire en $C_4$ à $C_{10}$ après la rétroscission de l'éther d'alkyle et de tertioalkyle en proportion de 60 à 99% en poids.

4. Procédé suivant les revendications 1 à 3, caractérisé en ce qu'on injecte dans le mélange d'hydrocarbures contenant au moins une oléfine tertiaire en $C_4$ à $C_{10}$, par mole d'oléfine tertiaire en $C_4$ à $C_{10}$ ou par mole de la somme molaire des diverses oléfines tertiaires en $C_4$ à $C_{10}$, 0,8 à 5 mol d'alcanol en $C_1$ à $C_4$ en partie sous forme d'alcanol en $C_1$ à $C_4$ récupéré par distillation et en partie sous forme d'alcanol en $C_1$ à $C_4$ obtenu par désorption de la résine absorbante.

5. Procédé suivant les revendications 1 à 4, caractérisé en ce que l'absorption de l'alcanol est effectuée à une température de 0 à 60°C et sous pression de 0,2 à 20 bar.

6. Procédé suivant les revendications 1 à 4, caractérisé en ce que la désorption de l'alcanol est conduite à 0-100°C et sous pression de 0,2 à 30 bar.

7. Procédé suivant les revendications 1 à 6, caractérisé en ce que la charge de la couche absorbante est réglée à 0,1-100 kg de courant chargé par kilogramme de matière absorbante par heure.